# EUROPEAN PATENT SPECIFICATION

(11) **EP 2 231 268 B1**
(45) Date of publication and mention of the grant of the patent: **26.02.2014**
(21) Application number: 08865509.7
(22) Date of filing: 19.12.2008
(51) Int. Cl.: A61N 1/36, A61N 1/372

(54) **MECHANICAL CONTROL OF ELECTRICAL NERVE STIMULATION SYSTEM FOR THE TREATMENT OF PELVIC DISORDERS**
MECHANISCHE STEUERUNG EINES ELEKTRISCHEN NERVENSTIMULATIONSSYSTEMS ZUR BEHANDLUNG VON BECKENERKRANKUNGEN
CONTRÔLE MÉCANIQUE DE SYSTÈME DE STIMULATION NERVEUSE ÉLECTRIQUE POUR LE TRAITEMENT DE TROUBLES PELVIENS

(30) Priority: 21.12.2007 EP 07150389; 21.12.2007 US 8559
(43) Date of publication of application: 29.09.2010
(73) Proprietor: Neurodan A/S, 9200 Aalborg SV (DK)
(72) Inventor: FJORBACK, Morten, DK-9260 Gistrup (DK); RIJKHOFF, Nico J. M., DK-9210 Aalborg Sø (DK); BORUP, Thomas, DK-9530 Støvring (DK); ERIKSEN, Michael, DK-9240 Nibe (DK)
(74) Representative: Inspicos A/S
(86) International application number: PCT/EP2008/068097
(87) International publication number: WO 2009/080786

(56) References cited:
- WO-A-2006/105245
- US-A- 3 236 240
- US-A1- 2004 172 087
- US-A1- 2006 122 660
- US-A1- 2006 190 051
- US-A1- 2007 239 224
- US-A1- 2007 255 085
- US-A1- 2007 270 921
- US-B1- 6 393 323
- ZHIDE TANG ET AL: "Transcutaneous Battery Recharging By Volume Conduction and its Circuit Modeling" ENGINEERING IN MEDICINE AND BIOLOGY SOCIETY, 2006. EMBS '06. 28TH ANNUAL INTERNATIONAL CONFERENCE OF THE IEEE, IEEE, PISCATAWAY, NJ, USA, 30 August 2006 (2006-08-30), pages 644-647, XP031235557 ISBN: 978-1-4244-0032-4
- YAO N ET AL: "Low Power Digital Communication in Implantable Devices Using Volume Conduction of Biological Tissues" ENGINEERING IN MEDICINE AND BIOLOGY SOCIETY, 2006. EMBS '06. 28TH ANNUAL INTERNATIONAL CONFERENCE OF THE IEEE, IEEE, PISCATAWAY, NJ, USA, 30 August 2006 (2006-08-30), pages 6249-6252, XP031339849 ISBN: 978-1-4244-0032-4

## Description

### Technical field

The present invention is generally concerned with the treatment of urge incontinence by electrical stimulation of pudendal nerve afferents, such as dorsal penile/clitoral nerves.

### Background of the invention

Electrical stimulation of pudendal nerve afferents is known to be effective in the treatment of a variety of pelvic disorders, such as urge urinary incontinence. The symptoms of increased daytime frequency, nocturia and urge urinary incontinence are often related to detrusor overactivity (DO), which is characterized by involuntary bladder contractions at low bladder volumes. The aforementioned symptoms adversely affect patients' quality of life due to social and hygienic difficulties. Upper urinary tract damage caused by sustained high intravesical pressures and repeated bladder infections is another concern that causes morbidity, hospitalization or even death in patients with DO. Bladder dysfunction is usually one of the most challenging long-term management issues in neurological patients because of the limited treatment options to help relieve symptoms. Approximately 1 million patients with symptoms of an overactive bladder (35% urge incontinence) are refractory to conventional treatment options world-wide. Especially neurological patients suffering from e.g. multiple sclerosis (MS), spinal cord injury (SCI), or stroke (CVA) are often difficult to treat by conventional means.

One way of electrically stimulating nerves is to convey electrical pulses to the nerves via an electrode implanted at or near a nerve or nerve afferent. Pulses are normally generated by implanted or external pulse generators, from which the electrical pulses are transmitted via an implanted or a percutaneous lead.

The pulse generator is typically programmable by means of an external control device, via wired or wireless data transmission. Normally pulses are delivered continuously or in predetermined cycles to achieve that the patient's urge to empty the bladder or bowel is suppressed for a period of time. However once the patient feels urgency, such urgency cannot be suppressed by the stimulation, and no warning is given to the patient, who may therefore not have sufficient time to reach a toilet.

US-A1-2007/239224 discloses a system according to the preamble of claim 1, including a non-implanted triggering mechanism for initiating stimulation.

### Summary of the invention

It is an object of embodiments of the present invention to provide a system and a method, which overcome the above disadvantages. It is a further object of embodiments of the present invention to provide a system and a method which allow for discrete use by the patient. If is a further object of embodiments of the invention to provide a system, which is convenient and safe in operation.

The invention provides a system according to claim 1.

The implantable battery provides the power for the pulses, once the pulse generator has been triggered. Thanks to the provision of the implantable battery, no external power supply for the implanted components is necessary. With a view to avoiding battery exchange by surgical intervention, the battery may be configured to receive wireless inductively transmitted charging current from an external charging device, providing inductive charging at a frequency below 315 kHz. Generally, for the purpose of charging a battery within the implantable pulse generator, wireless inductive signals should be provided at a frequency of below 300 kHz, preferably around 125 kHz.

The triggering period is comparatively very short compared to the stimulation period. In essence, triggering merely requires an activation signal which is transmittable in less than 1 second, whereas the stimulation period typically lasts for a predetermined period of approximately 1-2 minutes, unless stimulation is performed continuously, i.e. during periods of several hours.

Moreover, due to the provision of a mechanical triggering signal there is no requirement of a continuously listening receiver, and consequently less power is consumed. This in turn extends the life time of the implantable battery or, alternatively, allows for a smaller battery to be implanted.

The implantable user-operable triggering mechanism, which is operable by a mechanical impact to an outer surface portion of the patient's body, eliminates the need for an external triggering device to be worn or carried by the patient. Hence, the patient's handling of the system is facilitated, as those parts, which are required in the case of a need for immediate operation of the stimulation system, are all implanted/implantable.

The implantable triggering mechanism comprises a pressure, touch, force, sound or acceleration sensor for determining a user input. The pulse generator may be configured to set at least one stimulation parameter in dependence of said user input. For example, the mechanical triggering mechanism may be configured to allow the user to adjust stimulation amplitude. The sensor may e.g. be configured to determine the strength of the user pressure applied to the triggering mechanism and/or for determining the pace or speed of movement of the user's fingers across the triggering mechanism. Alternatively, the triggering mechanism may comprise a plurality of sensors to be placed at distinct subcutaneous positions, whereby the user input may be provided by activation of a selected onr of the sensors, or by activation of a predetermined combination of sensors. For example, simultaneous activation of two sensors may set a first value for a certain stimulation parameter, and activation of only one of the sensors may set a second value for that stimulation parameter.

The implanted/implantable triggering mechanism is configured to be activated in response to various types of mechanical impacts, such as pressure or acceleration, pressures/sound waves. Hence, the triggering mechanism may include at least one of: a mechanical transducer such as an accelerometer or/and a pressure switch, pressure sensor or pressure transducer.

In the present context, electrodes are said to be placed or placeable at, on, in, near or around nerves. It should be understood that any reference to the placement of electrodes is not limited to any specific of these locations. Hence, an electrode, which for example is said to be placed or placeable 'at' a nerve may be placed or placeable not only 'at', but also on, in, near or around that nerve, unless the present text specifically requires a certain location of the electrode. In general, the reference to any placement 'on', 'in', 'near' or 'around' nerves includes any placement 'at', 'on', 'in', 'near' or 'around'.

As used herein, the reference to pudendal nerve afferents includes the sensory part of the sacral nerves, dorsal penile/clitoral nerves (genital nerves), as well as any parts or branches thereof.

For safety reasons, the implantable triggering mechanism may include a plurality of sensors, so that only activation of more than one sensor provides a signal to generate pulses. Alternatively, the triggering mechanism may be provided to provide a signal for pulse generation only if the sensor is activated a plurality of times within a short period, e.g. by 'double-click' activation.

The triggering mechanism may be placed in the area of the stimulation electrode, i.e. close to the nerve or nerve branches to be stimulated, usually in the area of the pubic symphysis. However, users may prefer that the triggering mechanism is provided at a site remote from the pubic symphysis for reasons of discretion. Hence, the triggering mechanism may for example be provided in the chest region and operatively connected to the pulse generator via a subcutaneous wire or by wireless telemetry, such as inductive or radio frequency telemetry. Generally, the triggering mechanism and the pulse generator may be provided in separate housings, and the triggering mechanism may be configured to transmit the control signal to the pulse generator by wireless telemetry. In all respects, wireless data transmission from the triggering mechanism to the pulse generator may be provided through body tissue, preferably without inductive or radio frequency signals passing through ambient air.

Herein, inductive wireless signal are defined as signals transmitted at a frequency below 315 kHz. Radio frequency wireless signals are defined as signals transmitted at a frequency above 400 MHz.

Communication from the triggering mechanism to the pulse generator may be provided by means of body volume conduction, i.e. by using the body's conductivity for signal transmission, as described by Wegmueller M.S., IEEE 2006 "Galvanical coupling for data transmission through the human body" and IEEE 2007: "An attempt to model the human body as a communication channel".

The implanted/implantable battery may be provided as a separate, implantable component, or it may be provided within a housing of the triggering mechanism or within a housing of the pulse generator. Separate batteries may be provided for the triggering mechanism and the pulse generator, respectively, may be provided. The battery or batteries provide power for the control signal transmitted by the triggering mechanism, for the pulse generator and optionally also for the wireless transmission of signals from the implanted components to an external device, e.g. a control device.

In particular, the pudendal nerve branches, such as the genital nerves (penis nerve in men and clitoris nerve in women) may be stimulated by the system and method of the present invention. The invention is particularily useful for the treatment of urge urinary incontinence, but may also be applied in the treatment of faecal incontinence. Treatment of stress incontinence is also contemplated. Further, the invention may be useful for bladder voiding by stimulation of the perineal nerve afferents.

The system of the present invention is preferably event-triggered. In other words, stimulation may be initiated by a patient, once the patient feels a compelling desire to pass urine or faeces. The method of the present invention preferably provides direct sensory nerve stimulation to evoke a detrusor inhibitory reflex together with reflex mediated contraction of pelvic floor muscles that aids to preserve continence. In respect of applications, in which the electrode is implanted on, at or near a dorsal penile/clitoral nerve, stimulation only affects pudendal nerve afferents, i.e. stimulation is selectively targeted towards nerve afferents that affect the bladder. This is fundamentally different from systems, which provide continuous neuromodulaiton to modulate the spinal reflex arches, without allowing the patient to initiate stimulation. In such systems, urge to empty the bladder or bowel is suppressed for a period of time, however once the patient feels urgency, such urgency cannot be suppressed by the stimulation. Hence, no warning is given to the patient, who may therefore not have sufficient time to reach a toilet. The present invention provides a system that allows for patient-initiated stimulation. The patient will typically initiate stimulation once he or she feels a desire to empty the bladder or bowel, whereby the stimulation causes the urge to vanish for a period of time of e.g. 10 minutes, allowing the patient sufficient time to reach a toilet.

Though it is preferable that the patient may initiate stimulation for the above reasons, the systems and methods of the present invention may be configured to provide continuous stimulation under certain circumstances, e.g. when the patient is asleep. Alternatively, if the patient is physically capable of preserving continence when he or she is asleep, the system may be inactivated when the patient is asleep.

The at least one implantable electrode may include a cuff electrode, a wire, a helix, a double helix, a spiral electrode, or an intra-neural electrode. Preferably, the electrode is configured to be placed in the immediate vicinity of a pudendal nerve branch or around a nerve branch.

The pulse generator may include a receiver for receiving signals emitted by the triggering device. Preferably, the receiver is configured to receive electrical signals transmitted via an electrical lead from the triggering mechanism, so that it requires no power supply. The pulse generator may further include an electronic memory for storing stimulation programs and/or stimulation parameters. The electronic memory is preferably provided as a non-volatile memory, such as EPROM, EEPROM, flash memory or the like known per se. In order to program the pulse generator, the pulse generator and/or a receiver connected thereto may be configured to receive wireless transmitted signals from an external programming device. In one embodiment, the signals for programming may be provided as inductive signals, in which case the receiver for receiving the programming signals consumes no power other than power transmitted with the inductive signal. Embodiments relying on VHF/UHF signals for programming require a constantly listening receiver, which is to be powered by an implanted battery.

The external programming device for transmitting data to the pulse generator may be configured to transmit and receive said data via wireless radio communication or inductive telemetry. Generally, radio frequency signals have a wider range than inductive signals, and for certain purposes it may hence be beneficial with respect to user and programmer convenience that programming and other data transmission to the pulse generator is carried out by means of radio frequency signals.

The battery is preferably hermetically sealed within an implanted housing. For example, the battery may be provided in hermetically sealed compartment of a housing of the pulse generator and/or within a hermetically sealed compartment of a housing of the triggering mechanism.

The triggering mechanism may be user-controlled to allow the user to initiate pulse stimulation. Hence, stimulation may be event-triggered, e.g. following a compelling desire to pass urine or faeces. The triggering mechanism may include a programmable memory for storing stimulation programs and/or stimulation parameters, as well as appropriate data transmission elements for wireless programming of the triggering mechanism by an external programming device, and data transmission elements for passing stimulation programs and/or parameters to the pulse generator.

For applications, in which the physical dimensions of the pulse generator are of concern, i.e. applications, for which it is desired to minimize the dimensions of the pulse generators, it may be desirable to include as many components as possible in the vicinity of the triggering mechanism or within a housing of the triggering mechanism.

In preferred embodiments, the lead is detachably connected to the pulse generator, e.g. through a releasable connector. The lead may be permanently secured to the electrode or detachably coupled thereto. A further lead may be provided for subcutaneously connecting the triggering mechanism to the implanted pulse generator. The lead may be detachably or permanently secured to the triggering mechanism and the pulse generator, respectively.

In one embodiment the at least one implanted/implantable battery is rechargeable. For example, the battery may be rechargeable while it is left in place in the patient's body. In one embodiment the battery is inductively rechargeable, e.g. by means of an external charger. Alternatively, the battery may be removed by surgical intervention and recharged outside the patient's body. In other embodiments, the battery is non-rechargeable. In such cases the battery life time may exceed the expectation of the patient's remaining life time or the expected life time of the implanted parts of the product, or battery replacement may be performed by surgical intervention.

The pulse generator may be configured to provide the electrical pulses when it is in an active mode and to enter a dormant mode upon completion of a sequence of the pulses. The user-operable triggering mechanism may be configured to provide the inductive control signal with an amount of energy sufficient to cause the pulse generator to switch from the dormant mode to said active mode. By causing the pulse generator to enter the dormant mode, power consumption may be kept at a minimum level, in particular as the power required to cause the pulse generator to switch from the dormant mode to the active mode is provided by the triggering mechanism.

The dormant mode is to be understood as the time period between the event-triggered stimulation puls(es) has/have been sent from the pulse generator and the next user-operable triggering mechanism has been activated. Hence the duration of the dormant mode may vary during the day. In the dormant mode the stimulator's stimulation circuits consume very little or no power at all. Only a circuit for wakening up the entire stimulator consumes power.

In the present context, the term 'dormant mode' is hence not a mode, in which the pulse generator is completely disabled. To the contrary, the pulse generator is in a standby state, in which it is capable of detecting a mechanical impact for triggering the pulse generator. Thus, in this invention the dormant mode occurs several times during the day, i.e. following each and every time a pulse train has been delivered. The dormant mode accordingly occurs in cycles, the duration and frequency of which depend from the duration and frequency of user activation of the generation of pulses.

In the dormant mode, the pulse generator as such consumes no power. Preferably, a voltage is maintained only at a wake-up circuit, which is capable of causing the pulse generator to exit the dormant mode and enter the active mode in response to the mechanical impact to the outer surface of the user's body. In one embodiment, the system according to the present invention hence comprises:
- a wake-up circuit for providing said control signal to the pulse generator to cause it to exit the dormant mode and enter the active mode;
- at least one power source for powering the pulse generator and the wake-up circuit; wherein the power source is configured such that:
- a voltage is maintained at the wake-up circuit in the dormant mode of the pulse generator, and
- no voltage is maintained at the pulse generator in the dormant mode.

The wake-up circuit may be entirely housed or integrated in the pulse generator, or it may be provided as a separate implantable or external component. The power source may comprise one or more batteries, included e.g. in a housing of the pulse generator. Alternatively, the power source may include an external power source for transmitting power to the wake-up circuit and the pulse generator through wireless inductive transmission or by means of a percutaneous wire. In one embodiment, the system comprises an implantable battery for powering the wake-up circuit and a second external or implantable power source for powering the pulse generator, so as to reduce the frequency of battery exchange and/or battery recharge.

A voltage may be maintained at a circuit of the pulse generator other than the wake-up circuit, even in the dormant mode, in order to achieve fast wake up of the generator or in order to allow the pulse generator to perform certain functions in the dormant mode, e.g. low-battery check, hardware function tests etc.

Stimulation parameters, such as pulse voltage or current amplitude, pulse shape, pulse duration/width or frequency, may be pre-stored in a memory of the pulse generator.

The triggering mechanism may be configured to include at least one of the following commands in the control signal:
- a wake-up command for the implantable pulse generator;
- a command for the pulse generator to select a particular one of a plurality of stimulation programs pre-stored in a memory of the implantable pulse generator; and
- stimulation parameters of said stimulation pulses, including at least one of: an amplitude of the electrical pulses; a voltage of the electrical pulses; a pulse width of the electrical pulses; a pulse rate of the electrical pulses.

For example, the triggering mechanism may be configured to activate the pulse generator according to a first stimulation program, when a first mechanical impact is provided to the triggering mechanism, and to activate the pulse generator according to a second stimulation program, when a second mechanical impact is provided to the triggering mechanism. In one embodiment, a single pressure touch to the surface of the patient's body in the vicinity of the triggering mechanism may cause stimulation to be activated according to the first stimulation program, whereas a double pressure touch, i.e. to pressures applied within a short time frame, may cause stimulation to be activated according to the second stimulation program.

In some setups the system is programmed to provide a train of a predetermined number of pulses or to calculate the number of pulses (i.e. the duration of the train of pulses), whereby the train of pulses is determined by the control of the system, unless overruled by a patient-activated emergency stop. In other setups, it may however be desirable to allow the patient to prolong the stimulation period, whereby the system is programmed to generate electrical pulses until the patient stops the pulse generation, i.e. until the patient deactivates stimulation. In such embodiment, the system will typically be in its active mode for longer periods of time. Prolonged stimulation periods may in particular be useful for extending the patient's continence time immediately prior to bladder voiding, usually for a period of time of 1-5 minutes prior to voiding. User-activated termination of the train of pulses may also be beneficial in cases the patient reaches a toilet facility before expiration of a predetermined or pre-programmed train of pulses, so as to expedite bladder voiding. The possibility of user-activated termination of the pulse train also constitutes a safety feature, which may reduce excessive stimulation and/or reduce the patient's sensation of discomfort.

The level of charge of the electrical pulses may be controlled by controlling one or more of:
- the amplitude of current of the electrical stimulation pulses;
- the amplitude of voltage of the electrical stimulation pulses;
- the pulse rate of the electrical stimulation pulses, i.e. the length of intervals between the pulses;
- the pulse width of the electrical stimulation pulses;
- the shape of the electrical stimulation pulses, however square pulses are preferred.

The pre-stored stimulation programs may be configured to initiate the sequence of stimulation pulses by stepwise increasing charges of electrical pulses from an initial level to a final level. The stepwise increase of the pulse charges may be achieved by increasing current amplitude, voltage amplitude, pulse width or a combination thereof, or by stepwise increasing pulse frequency. One benefit of the stepwise increase of the pulse charges is that it decreases possible discomfort sensed by the patient at the start of stimulation. The gradual increase of the pulse charges may lead to higher patient comfort, not only during stimulation start-up, but also during the entire stimulation period.

A stepwise decrease of pulse charges from the final level back to the initial level (e.g. zero) following stimulation is also possible.

The stepwise increase of pulse charges (ramp up) may be provided as a user-selectable option. Hence, the user may for example select various stimulation programs, of which some provide stepwise increase of pulse charge, and some provide immediate stimulation at the final level without prior stepwise increase of the pulse charges. Likewise, the parameters of the stepwise pulse increase may be set according to a user selection. For example, the user may be allowed to choose ramp-up time, frequency, number of pulses to be provided during ramp-up etc.

The pulse generator may be configured to stepwise increase the pulse charges from the initial level to the final level during a period of time of about 0.1 seconds to about 40 seconds. The stepwise increase of the pulse charges may be linearly or exponentially increased.

The pulse generator is preferably configured to provide the pulses at a pulse rate of about 5 pulses per second to about 50 pulses per second. In preferred embodiments, the initial level provides no current, whereas the final level provides charges at a current from about 0.01 mA to about 100 mA, such as from about 0.01 mA to about 10 or 20 mA. The at least one electrode is preferably configured to be placed at, on, in, near or around a genital nerve, such as a dorsal penile/clitoral nerve.

The pulses are typically provided at a pulse width of about 50 µs to about 400 µs, such as at a pulse width of about 200 µs.

The at least one electrode is implanted or implantable at a left and/or right branch of the dorsal penile/clitoral nerve, which is a distal portion of the pudendal nerve close to the genitals. The electrode may be implanted at or near the genitals or in Alcock's Canal via the perineum.

The pulse generator may be implantable/implanted in a subcutaneous pocket in the pelvic or abdominal region at a tissue depth of approximately 4 cm or less. In other embodiments, the pulse generator may be implantable/implanted to a tissue depth of between 0.3 cm and 0.4 cm or between 0.6 cm and 1.4 cm, or between 1.6 cm and 1.9 cm or between 2.1 and 2.8 cm. The pulse generator is preferably implanted in a subcutaneous pocket in the pelvic or abdominal region, such as in the anterior pelvic or abdominal region. The implant may contain means for fixation, e.g. sutures.

In order to avoid tissue damage, the implantable pulse generator may be configured to control stimulation parameters of the stimulation pulses and to charge balance the pulses. Charge balancing may be achieved by alternating polarity, by shortcircuiting the electrodes after each stimulation pulse, through a capacitor coupled in series with the electrodes, or by software-controlled means.

In order to allow the implanted pulse generator and/or the implanted triggering mechanism to communicate to an external device, the pulse generator and/or the triggering mechanism may comprise a signal receiver for receiving a wireless signal from the implantable pulse generator. The implantable triggering mechanism and/or pulse generator may in such an embodiment be configured to transmit a wireless signal to the external device, e.g. in case of one or more predetermined conditions of the implantable components, such as upon detection of a low battery charge. In one embodiment, the pulse generator is configured only to communicate a low battery status. In other embodiments, other information may be transmitted by the implanted pulse generator, such as information about malfunctions in the implanted components.

The triggering mechanism may be configured to transmit stimulation parameters to the implantable pulse generator via an electrical lead or wireless communication means. Such stimulation parameters may include at least one of a current of the electrical pulses, e.g. a current amplitude, a voltage of the electrical pulses, a pulse width of the electrical pulses, and a frequency of the electrical pulses.

An external programming device may be provided in order to transmit the pulse stimulation parameters or pulse generation programs to the implantable pulse generator and/or to the implanted triggering mechanism. The external programming device may be at the disposal of the patient, or it may alternatively be provided at a medical facility, operable by a trained physician only. Preferably, the external programming device includes suitable software, user interface, memory, power unit, and data processor.

The implantable leads may be helical in order to provide a flexible and mechanically strong connection. The leads may be coated with a biocompatible material or made entirely from a biocompatible material.

The housing of the pulse generator and triggering mechanism is preferably coated with or made from a biocompatible material, such as titanium.

The invention also provides a surgical kit for preparing a system according to the invention.

The electrode may be connected to the lead, thereby alleviating surgical personnel from the task of connecting the electrode to the lead. A tool for connecting the lead to the pulse generator may be included in the kit. The kit may further include tools for implanting at least one of the electrode, the lead and the pulse generator, e.g. for tunneling the lead through body tissue.

In embodiments relying on wired signal transmission from the triggering mechanism to the pulse generator, the kit preferably also includes a second lead for connecting the triggering mechanism to the pulse generator. The lead may be detachably or permanently secured to the triggering mechanism and the pulse generator, respectively.

### Description of the drawings

Embodiments of the invention will be described with reference to the accompanying drawings, in which:
Fig. 1 shows an illustration of the pelvic region of a patient, including an implanted electrode, pulse generator and lead, as well as an external triggering mechanism;
Fig. 2 shows an illustration of the pulse generator shown in Fig. 1;
Figs. 3 and 4 are illustrations of an implanted triggering mechanism, which is activatable by mechanical impact to an outer surface portion of the patient's body;
Figs. 5 and 6 are illustrations of a wireless external programming device;
Fig. 7 is an anatomic view showing the implanted pulse generator of Figs. 1 and 2 in association with a clinical programming system;
Fig. 8 is a flow chart of processes of the system of the present invention;
Figs. 9 and 10 are graphical representations of biphasic stimulus current outputs from the pulse generator;
Fig. 11 is an illustration of a sterilized kit including implantable elements as well as surgical tools for their implantation;
Fig. 12 illustrates a releasable connection between a pulse generator and the lead connecting the pulse generator to the electrode.

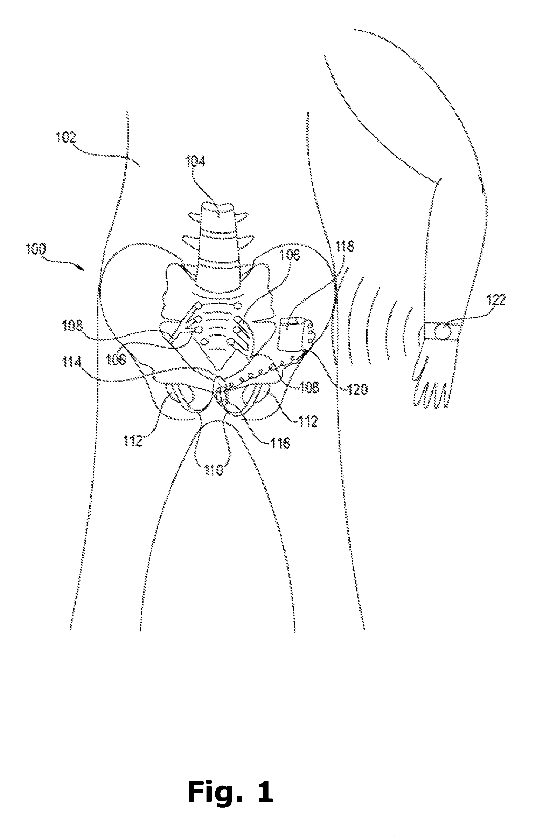

Fig. 1 generally illustrates the pelvis 100 of a patient 102 and a portion of the spinal cord 104. The pudendal nerves 108 exit the sacral cord and branch into the dorsal penile/clitoral nerves 110. Fig. 1 further illustrates Alcock's Canal 112 and the pubic symphysis 114. In the illustration of Fig. 1, a cuff electrode 116 is placed around the left dorsal penile/clitoral nerve 110. The electrode 116 is connected to an implanted pulse generator 118 via an implanted lead 120. The pulse generator 118 comprises a battery (not shown in Fig. 1) and is configured to convey pulses of electrical current through the electrode 116 via the lead 120, so as to provide electrical stimulation to the dorsal penile/clitoral nerve 110. The battery may be chargeable by means of inductive wireless power transmission providing electromagnetic signals at a frequency of below 315 kHz.

An external, wrist worn device 122 is provided for wirelessly communicating with the pulse generator 118. The wrist worn device 122 is configured as a programming device for setting stimulation parameters or for otherwise programming or configuring the implanted pulse generator 118. The pulse generator 118 may be configured for providing data to the external device 122, such as e.g. a low battery warning of the pulse generator 118.

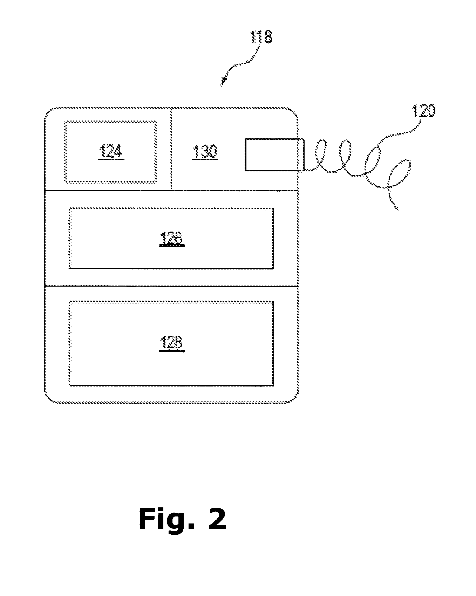

Fig. 2 generally shows the main components of the pulse generator 118. The pulse generator includes a telemetry unit 124 for receiving and/or sending information to/from an external device, such as the wrist worn device 122 shown in Fig. 1. Further, the pulse generator includes a pulse generating circuitry 126, and a battery 128. The battery 128 is preferably hermetically sealed within a housing of the pulse generator 118. The pulse generator contains a connector 130 to attach the implantable lead 120.

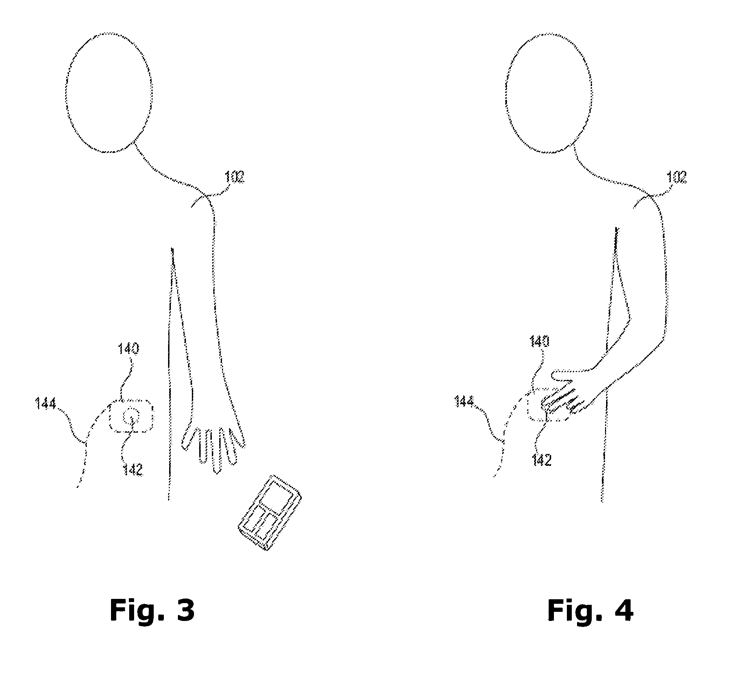

Figs. 3 and 4 are illustrations of an implanted triggering mechanism 140. The triggering mechanism is activatable by mechanical impact to an outer surface portion of the patient's body, e.g. by a push button 142, which is activatable by application of a finger pressure to the outer surface portion of the body, as illustrated in Fig. 4. The triggering mechanism 140 may also house the pulse generator, in which case a distal end of the lead 144 is connected to the electrode (not shown). Alternatively, the pulse generator may be provided as a separately implanted component, whereby the lead 144 interconnects the triggering mechanism and the pulse generator. An external programming device 146 is provided for programming or configuring the implanted pulse generator and/or the implanted triggering mechanism 140 via wireless data transmission.

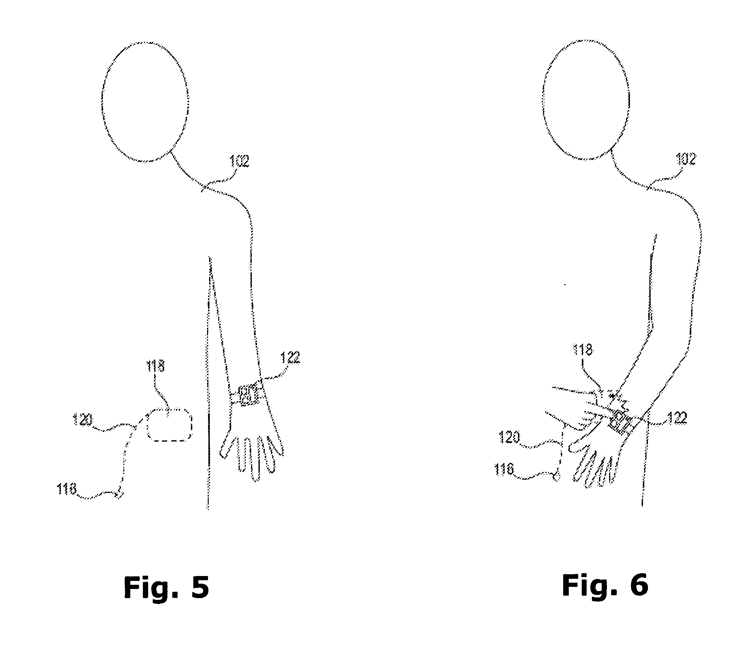

As mentioned above, the wrist worn device 122 may serve as a wireless external programming device for programming or configuring the pulse generator. As shown in Figs. 5 and 6, the patient 102 may program the pulse generator 118 by locating the wrist worn device 122 in the vicinity of the implanted pulse generator 118 and activating a programming action, so as to wirelessly transmit appropriate programming or configuration data to the pulse generator 118, as shown in Fig. 6.

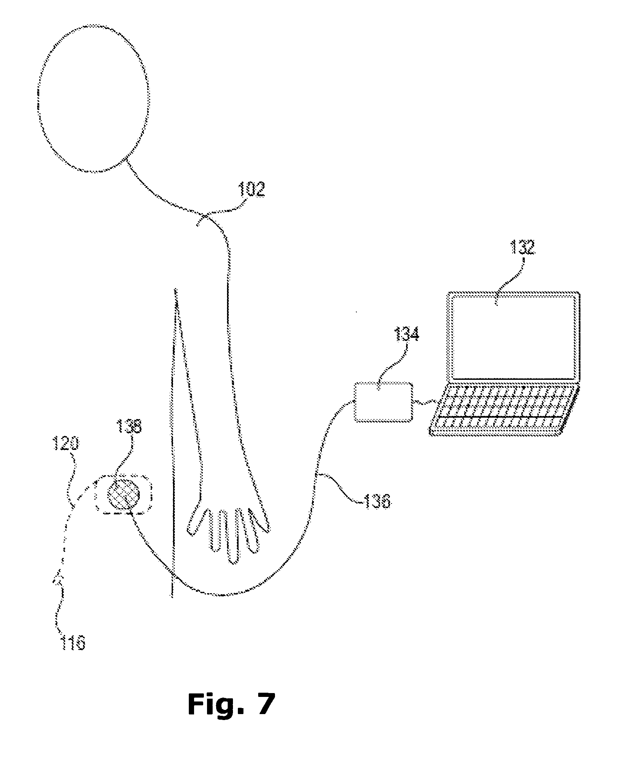

Fig. 7 illustrates a clinical programming system for a system according to the invention. The programming system includes a personal computer 132, which is typically operated by a physician, and a programming box 134. The programming box is connected to an inductive coil 138 via a lead 136. Programming of the implanted pulse generator 118 is performed via wireless, inductive telemetry from the inductive coil through the skin of the patient.

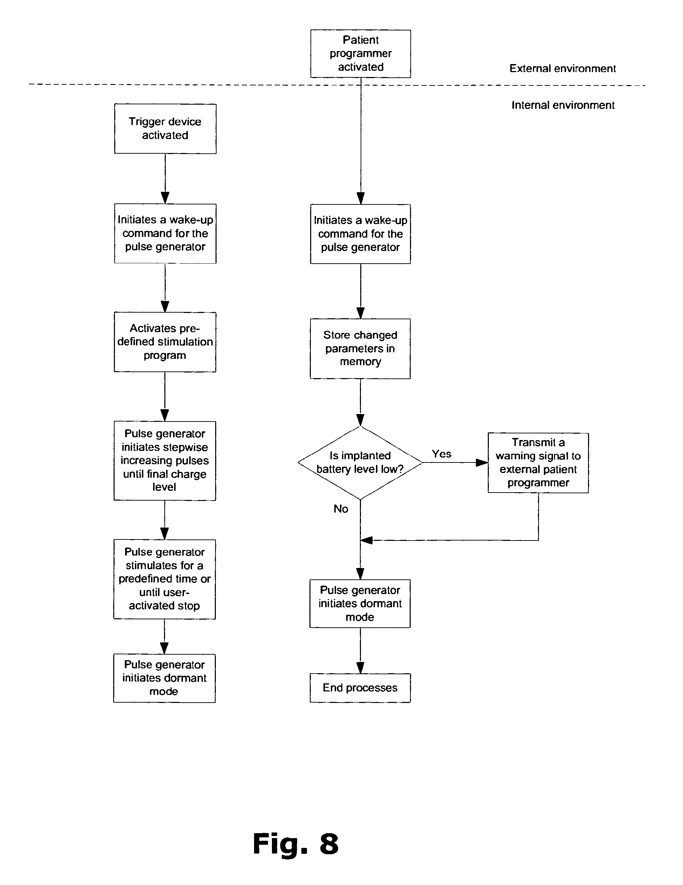

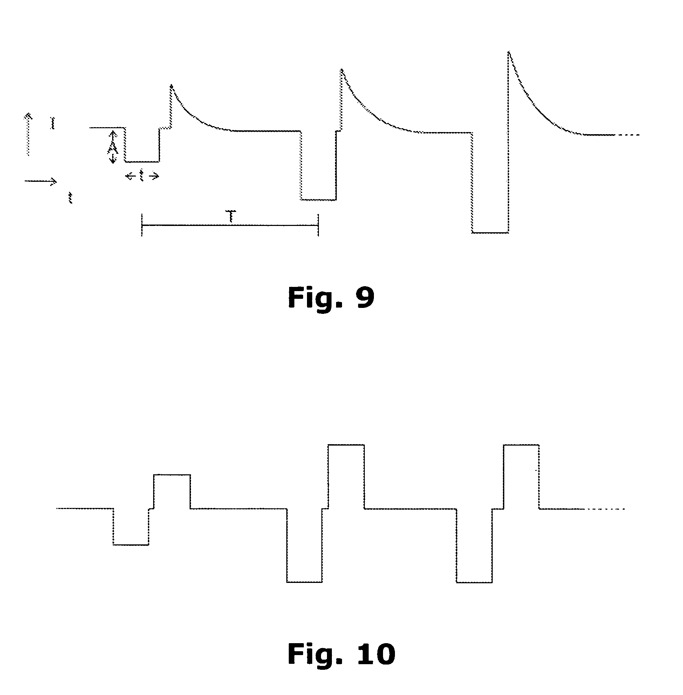

Fig. 8 shows a flow chart of a process occurring in the system of Figs. 8 and 9, relying on a triggering mechanism, which is activatable upon application of a mechanical impact to an outer surface portion of the patient's body.

In the left part of Fig. 8, the pulse generator is initially in a dormant mode. When the patient activates the trigger device (triggering mechanism) 140, a trigger signal is provided to initiate a wake-up command for the pulse generator. The wake-up command causes the pulse generator to enter an active mode and to activate a predefined stimulation program. The pulse generator then initiates a stepwise increase of pulse charges from zero up to a final charge level, and pulse stimuli are provided for a predetermined period of time, or until the patient deactivates the pulses. Finally, the pulse generator enters a dormant mode.

The right part of Fig. 8 illustrates a programming process for the system of Figs. 1-7. When a patient or physician programmer is activated, a wake-up command for the pulse generator is initiated. Changed parameters are then stored in a memory of the pulse generator, and a warning signal is transmitted to the external patient programmer if a low battery level is detected.

Figs. 9 and 10 are graphical representations of biphasic stimulus current outputs from the pulse generator during initial stepwise increase of the pulse charges upon activation of the pulse generator. In the example shown, the current amplitudes A of the pulse are stepwise increased. It should however be understood that the pulse charges may also be increased by extending the pulse width t, shortening the intervals between the pulses T, or stepwise increase the pulse voltages. The chart of Fig. 9 illustrates de-charge of a series capacitor upon short-circuiting of electrodes. In the example of Fig. 10, alternating polarity is achieved by alternating the current through the electrode by means of hardware or software control embedded in the pulse generator.

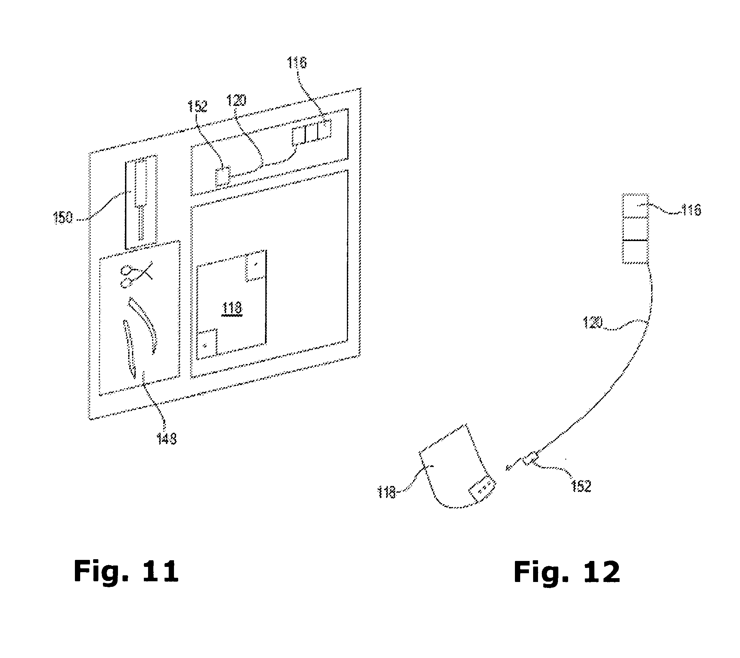

Fig. 11 illustrates an embodiment of a sterilized surgical kit. The kit includes several sterilized packages. One package contains the pulse generator 118. Another package contains the electrode 116, connected to a connector 152 via the lead 120. Further packages 148 and 150 include surgical tools for implanting the electrode 116, pulse generator 118 and lead 120. The surgical tools may for example include tunneling tools for tunneling the lead and/or electrode through the patient's body during surgical intervention. As shown in Fig. 12, the connector 152 allows the lead 120 to be releasably connected to the pulse generator 118.

## Claims

1. A system for the treatment of urge incontinence by electrical stimulation of pudendal nerve afferents of a living being, including:
- an implantable electrode (116) configured to be placed on, at, in, around or near a portion of a pudendal nerve or its branches;
- an implantable pulse generator (118) having a pulse-generating circuitry for providing electrical pulses to the electrode (116) in order to achieve said electrical stimulation of the pudendal nerve (108) or its branches (110), the implantable pulse generator (118) being configured to receive control signals and to provide said electrical pulses in response to said control signals;
- an implantable battery (128) for powering the pulse generator (118);
- an electrical lead (120;144) for connecting the pulse generator (118) to the electrode (116); **characterized by**
- an implantable user-operable triggering mechanism (140) configured to transmit a control signal to the implantable pulse generator (118) in order to trigger a sequence of said electrical pulses, the implantable user-operable triggering mechanism (140) being configured to transmit said control signal in response to a mechanical impact to an outer surface portion of the user's (102) body, the triggering mechanism (140) comprising at least one pressure, touch, force, sound or acceleration sensor (142) for determining a user input and being configured to transmit said control signal only:
- if the sensor is activated a plurality of times within a short period, or
- at a given strength of user pressure, or
- at a given pace or speed of movement of the user's (102) fingers across the triggering mechanism (140), or
- at activation of a selected one of a plurality of sensors at distinct subcutaneous positions,
- or by activation of a predetermined combination of sensors.

2. A system according to claim 1, wherein the battery (128) is configured to receive wireless inductively transmitted charging current from an external charging device.

3. A system according to any of the preceding claims, wherein the implantable triggering mechanism (140) comprises a sensor (142) for determining a user input, and wherein the pulse generator (118) is configured to set at least one stimulation parameter in dependence of said user input.

4. A system according to any of the preceding claims, further comprising an implantable mechanical sensor (142) configured to detect said mechanical impact.

5. A system according to any of the preceding claims, wherein the battery (128) is rechargeable.

6. A system according to any of the preceding claims, wherein the pulse generator (118) is configured to provide said electrical pulses when it is in an active mode, the pulse generator (118) being further configured to enter a dormant mode upon completion of a sequence of said pulses.

7. A system according to any of the preceding claims, wherein the triggering mechanism (140) is configured to include at least one of the following commands in the control signal:
- a wake-up command for the implantable pulse generator;
- a command for the pulse generator to select a particular one of a plurality of stimulation programs pre-stored in a memory of the implantable pulse generator; and
- stimulation parameters of said stimulation pulses, including at least one of: an amplitude of the electrical pulses; a voltage of the electrical pulses; a pulse width of the electrical pulses; a pulse rate of the electrical pulses.

8. A system according to claim 7, wherein said pre-stored stimulation programs are configured to initiate said sequence of stimulation pulses by stepwise increasing charges of electrical pulses from an initial level to a final level.

9. A system according to any of the preceding claims, wherein the implantable pulse generator (118) is configured to control stimulation parameters of said stimulation pulses and to charge balance the pulses.

10. A system according to any of the preceding claims, wherein the triggering mechanism (140) comprises a signal receiver for receiving a wireless signal from the implantable pulse generator (118), and wherein the implantable pulse generator (118) is configured to transmit a wireless signal to the triggering mechanism (140) in case of one or more predetermined conditions of the implantable components.

11. A system according to any of the preceding claims, wherein the triggering mechanism (140) comprises a memory for storing stimulation parameters.

12. A system according to any of the preceding claims, wherein the triggering mechanism (140) is configured to transmit said control signal and/or stimulation parameters by body volume conduction.

13. A system according to any of the preceding claims, further comprising an external programming device (132; 134) configured to transmit pulse stimulation parameters or pulse generations programs to the implantable pulse generator (118).

14. A surgical kit for preparing a system according to any of claims 1-13 for the treatment of urge incontinence by electrical stimulation of pudendal nerve afferents of a living being, wherein the electrode, the pulse generator and the lead according to claim 1 are packed in a sterile condition.

## Patentansprüche

1. System zur Behandlung einer Dranginkontinenz durch elektrische Stimulation von Nervus-pudendus-Afferenzen eines Lebewesens, wobei das System Folgendes umfasst:
- eine implantierbare Elektrode (116), die dafür konfiguriert ist, auf, an, in, um oder nahe einem Abschnitt eines Nervus pudendus oder seiner Zweige angeordnet zu werden,
- einen implantierbaren Impulserzeuger (118), der Impulserzeugungsschaltungen hat, um elektrische Impulse für die Elektrode (116) bereitzustellen, um die elektrische Stimulation des Nervus pudendus (108) oder seiner Zweige (110) zu erreichen, wobei der implantierbare Impulserzeuger (118) dafür konfiguriert ist, Steuersignale zu empfangen und die elektrischen Impulse als Reaktion auf die Steuersignale bereitzustellen,
- eine implantierbare Batterie (128), um den Impulserzeuger (118) zu speisen,
- eine elektrische Leitung (120; 144), um den Impulserzeuger (118) mit der Elektrode (116) zu verbinden, **gekennzeichnet durch**
- einen implantierbaren anwenderbedienbaren Auslösemechanismus (140), der dafür konfiguriert ist, ein Steuersignal an den implantierbaren Impulserzeuger (118) zu übermitteln, um eine Folge der elektrischen Impulse auszulösen, wobei der implantierbare anwenderbedienbare Auslösemechanismus (140) dafür konfiguriert ist, das Steuersignal als Reaktion auf einen mechanischen Anstoß an einen Außenflächenabschnitt des Körpers des Anwenders (102) zu übermitteln, wobei der Auslösemechanismus (140) wenigstens einen Druck-, Berührungs-, Schall- oder Beschleunigungssensor (142) umfasst, um eine Anwendereingabe festzustellen, und dafür konfiguriert ist, das Steuersignal nur zu übertragen:
- falls der Sensor mehrere Male innerhalb eines kurzen Zeitraums aktiviert wird oder
- bei einer gegebenen Stärke des Anwenderdrucks oder
- bei einem/einer gegebenen Bewegungstempo oder -geschwindigkeit der Finger des Anwenders (102) über den Auslösemechanismus (140) oder
- bei einer Aktivierung eines ausgewählten von mehreren Sensoren an unterschiedlichen subkutanen Positionen
- oder **durch** eine Aktivierung einer vorbestimmten Kombination von Sensoren.

2. System nach Anspruch 1, wobei die Batterie (128) dafür konfiguriert ist, einen drahtlos induktiv übermittelten Ladestrom von einer äußeren Ladeeinrichtung zu empfangen.

3. System nach einem der vorhergehenden Ansprüche, wobei der implantierbare Auslösemechanismus (140) einen Sensor (142) umfasst, um eine Anwendereingabe festzustellen, und wobei der Impulserzeuger (118) dafür konfiguriert ist, wenigstens einen Stimulationsparameter in Abhängigkeit von der Anwendereingabe festzusetzen.

4. System nach einem der vorhergehenden Ansprüche, das ferner einen implantierbaren mechanischen Sensor (142) umfasst, der dafür konfiguriert ist, den mechanischen Anstoß zu erfassen.

5. System nach einem der vorhergehenden Ansprüche, wobei die Batterie (128) wiederaufladbar ist.

6. System nach einem der vorhergehenden Ansprüche, wobei der Impulserzeuger (118) dafür konfiguriert ist, die elektrischen Impulse bereitzustellen, wenn er sich in einem aktiven Modus befindet, wobei der Impulserzeuger (118) ferner dafür konfiguriert ist, nach dem Abschluss einer Folge der Impulse in einen Ruhemodus einzutreten.

7. System nach einem der vorhergehenden Ansprüche, wobei der Auslösemechanismus (140) dafür konfiguriert ist, wenigstens einen der folgenden Befehle in dem Steuersignal einzuschließen:
- einen Weckbefehl für den implantierbaren Impulserzeuger,
- einen Befehl, damit der Impulserzeuger ein bestimmtes von mehreren Stimulationsprogrammen auswählt, die in einem Speicher des implantierbaren Impulserzeugers vorgespeichert sind, und
- Stimulationsparameter der Stimulationsimpulse, die wenigstens einen der folgenden einschließen: eine Amplitude der elektrischen Impulse, eine Spannung der elektrischen Impulse, eine Impulsbreite der elektrischen Impulse, eine Impulsfrequenz der elektrischen Impulse.

8. System nach Anspruch 7, wobei die vorgespeicherten Stimulationsprogramme dafür konfiguriert sind, die Folge von Stimulationsimpulsen einzuleiten durch ein schrittweises Steigern der Ladungen von elektrischen Impulsen von einem anfänglichen Niveau bis zu einem abschließenden Niveau.

9. System nach einem der vorhergehenden Ansprüche, wobei der implantierbare Impulserzeuger (118) dafür konfiguriert ist, Stimulationsparameter der Stimulationsimpulse zu regeln und die Ladung der Impulse auszugleichen.

10. System nach einem der vorhergehenden Ansprüche, wobei der Auslösemechanismus (140) einen Signalempfänger umfasst, um ein drahtloses Signal von dem implantierbaren Impulserzeuger (118) zu empfangen, und wobei der implantierbare Impulserzeuger (118) dafür konfiguriert ist, im Fall eines oder mehrerer vorbestimmter Zustände der implantierbaren Komponenten ein drahtloses Signal an den Auslösemechanismus (140) zu übermitteln.

11. System nach einem der vorhergehenden Ansprüche, wobei der Auslösemechanismus (140) einen Speicher zum Speichern von Stimulationsparametern umfasst.

12. System nach einem der vorhergehenden Ansprüche, wobei der Auslösemechanismus (140) dafür konfiguriert ist, das Steuersignal und/oder die Stimulationsparameter durch Körpervolumenleitung zu übermitteln.

13. System nach einem der vorhergehenden Ansprüche, das ferner eine äußere Programmierungseinrichtung (132; 134) umfasst, die dafür konfiguriert ist, Impulsstimulationsparameter oder Impulserzeugungsprogramme an den implantierbaren Impulserzeuger (118) zu übermitteln.

14. Chirurgisches Besteck zum Vorbereiten eines Systems nach einem der Ansprüche 1 bis 13 zur Behandlung einer Dranginkontinenz durch elektrische Stimulation von Nervus-pudendus-Afferenzen eines Lebewesens, wobei die Elektrode, der Impulserzeuger und die Leitung nach Anspruch 1 in einem sterilen Zustand verpackt sind.

## Revendications

1. Système de traitement d'une incontinence impérieuse par stimulation électrique des afférences du nerf pudendal d'un être vivant, comprenant :
- au moins une électrode implantable (116) configurée pour être placée sur, dans, autour ou près d'une partie du nerf pudendal ou de ses ramifications ;
- un générateur d'impulsions (118) possédant un circuit de génération d'impulsions pour fournir des impulsions électriques à l'électrode (116) afin d'obtenir la stimulation électrique du nerf pudendal (108) ou de ses afférences (110), le générateur d'impulsion implantable (118) étant configuré pour recevoir des signaux de contrôle et fournir lesdites impulsions électriques en réponse auxdits signaux de contrôle ;
- une batterie implantable (128) pour alimenter le générateur d'impulsions (118) ;
- un conducteur électrique (120 ; 144) pour connecter le générateur d'impulsions (118) à l'électrode (116) ; **caractérisé par**
- un mécanisme de déclenchement implantable actionnable par l'utilisateur (140) configuré pour transmettre un signal de commande au générateur d'impulsions implantable (118) pour déclencher une séquence d'impulsions électriques, le mécanisme de déclenchement actionnable par l'utilisateur implantable (140) étant configuré pour transmettre ledit signal de commande en réponse à un choc mécanique à une partie de surface externe du corps de l'utilisateur (102), le mécanisme de déclenchement (140) comprenant au moins une pression, un toucher, une force, un son ou un capteur d'accélération (142) pour déterminer une entrée utilisateur et étant configuré pour transmettre ledit signal de commande uniquement :
- si le capteur est activé plusieurs fois pendant une période courte, ou
- à une force donnée de pression de l'utilisateur, ou
- à un rythme ou vitesse de déplacement donné des doigts de l'utilisateur (102) à travers le mécanisme de déclenchement (140), ou
- lors de l'activation d'un capteur choisi parmi une pluralité de capteurs à des positions sous-cutanées distinctes, ou
- par activation d'une combinaison prédéterminée de capteurs.

2. Système selon la revendication 1, dans lequel la batterie (128) est configurée pour recevoir un courant de charge sans fil par induction transmis à partir d'un dispositif de charge externe.

3. Système selon l'une quelconque des revendications précédentes, dans lequel le mécanisme de déclenchement implantable (140) comprend un capteur (142) pour déterminer une entrée utilisateur, et dans lequel le générateur d'impulsions (118) est configuré pour régler au moins un paramètre de stimulation en fonction de ladite entrée utilisateur.

4. Système selon l'une quelconque des revendications précédentes, comprenant en outre un capteur mécanique implantable (142) configuré pour déterminer ledit impact mécanique.

5. Système selon l'une quelconque des revendications précédentes, dans lequel la batterie (128) est rechargeable.

6. Système selon l'une quelconque des revendications précédentes, dans lequel le générateur d'impulsions (118) est configuré pour fournir lesdites impulsions électriques lorsqu'il est en mode actif, le générateur d'impulsions (118) étant en outre configuré pour se mettre en mode inactif après achèvement d'une séquence desdites impulsions.

7. Système selon l'une quelconque des revendications précédentes, dans lequel le mécanisme de déclenchement (140) est configuré pour inclure l'une des commandes suivantes dans le signal de commande :
- une commande de réveil pour le générateur d'impulsions implantable ;
- une commande pour le générateur d'impulsions pour sélectionner un programme de stimulation particulier parmi une pluralité de programmes de stimulation pré-mémorisée dans une mémoire du générateur d'impulsions implantable ; et
- des paramètres de stimulation desdites impulsions de stimulation, incluant au moins : une amplitude d'impulsions électriques ; une tension d'impulsions électriques ; une largeur d'impulsion des impulsions électriques ; un taux d'impulsion des impulsions électriques.

8. Système selon la revendication 7, dans lequel les programmes de stimulation pré-stockés sont configurés pour initier ladite séquence d'impulsions de stimulation en augmentant progressivement les charges des impulsions électriques d'un niveau initial à un niveau final.

9. Système selon l'une quelconque des revendications précédentes, dans le générateur d'impulsions implantable (118) est configuré pour contrôler les paramètres de stimulation desdites impulsions de stimulation et pour l'équilibre de charge des impulsions.

10. Système selon l'une quelconque des revendications précédentes, dans lequel le mécanisme de déclenchement (140) comprend un récepteur de signal pour recevoir un signal sans fil du générateur d'impulsions implantable (118), et dans lequel le générateur d'impulsions implantable (118) est configuré pour transmettre un signal sans fil au mécanisme de déclenchement (140) dans le cas d'une ou plusieurs conditions prédéterminées des composants implantables.

11. Système selon l'une quelconque des revendications précédentes, dans lequel le mécanisme de déclenchement (140) comprend une mémoire pour stocker les paramètres de stimulation.

12. Système selon l'une quelconque des revendications précédentes, dans lequel le mécanisme de déclenchement (140) est configuré pour transmettre ledit signal de commande et/ou les paramètres de stimulation par conduction du volume corporel.

13. Système selon l'une quelconque des revendications précédentes, comprenant un dispositif de programmation externe (132 ; 134) configure pour transmettre des paramètres de stimulation d'impulsions ou des programmes de génération d'impulsions au générateur d'impulsions implantable (118).

14. Kit chirurgical pour préparer un système conformément à l'une quelconque des revendications 1 à 13 pour le traitement d'une incontinence impérieuse par stimulation électrique des afférences du nerf pudendal d'un être vivant, dans lequel l'électrode, le générateur d'impulsions et le conducteur, conformément à la revendication 1, sont emballés dans des conditions stériles.
